# EUROPEAN PATENT SPECIFICATION

(11) **EP 2 877 169 B1**
(45) Date of publication and mention of the grant of the patent: **11.10.2017**
(21) Application number: 12766715.2
(22) Date of filing: 27.07.2012
(51) Int. Cl.: A61K 31/20, A61K 31/202, A61K 35/20, A61P 17/00, A61K 35/00, A61P 17/02

(54) **PHARMACEUTICAL COMPOSITION OF MILK FAT GLOBULES (MFGs) ZINC FREE**
ZINKFREIE PHARMAZEUTISCHE ZUSAMMENSETZUNG AUS MILCHFETTGLOBULI
COMPOSITION PHARMACEUTIQUE DE GLOBULES DE MATIÈRE GRASSE DU LAIT (MFG) DÉPOURVUE DE ZINC

(43) Date of publication of application: 03.06.2015
(73) Proprietor: Fardoussi, Kassem Khal, Aleppo (SY)
(72) Inventor: Fardoussi, Kassem Khal, Aleppo (SY)
(74) Representative: Ganguillet, Cyril
(86) International application number: PCT/IB2012/053859
(87) International publication number: WO 2014/016647

(56) References cited:
- EP-A1- 1 319 407
- WO-A2-2008/140335
- ARGOV N ET AL: "Milk fat globule structure and function: nanoscience comes to milk production", TRENDS IN FOOD SCIENCE AND TECHNOLOGY, ELSEVIER SCIENCE PUBLISHERS, GB, vol. 19, no. 12, 1 December 2008 (2008-12-01), pages 617-623, XP025695187, ISSN: 0924-2244, DOI: 10.1016/J.TIFS.2008.07.006 [retrieved on 2008-07-25]
- ZAVA S ET AL: "Mare's Colostrum Globules Stimulate Fibroblast Growth In Vitro: A Biochemical Study", JOURNAL OF MEDICINAL FOOD, MARY ANN LIEBERT, LARCHMONT, NY, US, vol. 12, no. 4, 7 September 2009 (2009-09-07), pages 836-845, XP002652466, ISSN: 1096-620X, DOI: 10.1089/JMF.2008.0139
- PRASAD V ET AL: "Evaluation of ghee based formulation for wound healing activity", JOURNAL OF ETHNOPHARMACOLOGY, ELSEVIER IRELAND LTD, IE, vol. 107, no. 1, 11 August 2006 (2006-08-11), pages 38-47, XP027939612, ISSN: 0378-8741 [retrieved on 2006-08-11]

## Description

### Technical Field

The present invention concerns pharmaceutical compositions for use in treating various diseases relative to squamous cell carcinoma, skin burns, infections of skin wounds and burn wounds by pseudomonas aeruginosa. Among the targeted skin disorders are skin wounds, crust formation, hypertrophic scar formation of full-thickness skin wound, keloids.

The invention concerns in particular pharmaceutical compositions comprising natural products, namely Milk Fat Globules (MFG), and being free of zinc oxide for use in the treatment of the above diseases and disorders.

### Prior Art and the Problem Underlying the Invention

Cancer is one of the leading causes of death in developed countries. Cancer is a class of diseases characterized by two heritable properties: uncontrolled cell division and the ability of these cells to invade other tissues, either by direct growth into adjacent tissue (invasion) or by migration of cells to distant sites (metastasis). The hyper-proliferative properties initially give rise to a tumor or neoplasm. The unregulated growth is caused by damaged DNA, resulting in mutations to vital genes that control cell division, the cell cycle, among other functions. Cancers can be classified according to the tissue and cell type from which they arise. Cancers developing from epithelial cells are called carcinomas, and those from connective and muscle cells are called sarcomas. Additional cancers include those arising from hematopoietic cells (e.g., leukemia) and cancers of the nervous system.

In general, cancers appear to arise during a process in which an initial population of abnormal cells evolves into more aberrant cells through successive cycles of mutation and selection. More than 100 different genes have been identified which, when mutant, result in cancer. These so-called cancer-critical genes include growth factors, cytokines, hormones, extracellular matrix, etc., which are involved in many signaling pathways. Thus cancer-mediated activation of the coagulation system plays an important role in tumor-related activities such as growth and metastasis, and clinically, cancer patient with thrombosis have reduced life expectancy when compared to cancer's patients without thrombosis. Further it is shown that arachidonic acid induces cancer cell growth and proliferation in vitro. The non-exhaustive list of potential cancers involved in this process is colon, pancreatic, breast, prostate, lung, skin, urinary bladder, liver, colorectal, esophageal, breast, lung, and bladder cancers. It is also demonstrated that chronic inflammation, bacterial and viral infections (e.g., Helicobacter hepaticus, Salmonella typhimurium, Hepatitis virus C) play a role in the development of particular cancers such as hepatic cancer, gastric adenocarcinoma. Moreover cancer pain significantly affects the diagnosis, quality of life and survival of patients with cancer.

Oral cancer such as squamous cell carcinoma (SCC), common malignancy worldwide, is an important contributor to cancer morbidity and mortality and to overall international cancer burden. Squamous cell carcinoma is a type of non-melanoma skin cancer and also occurs as a form of cancer in diverse tissues, including the lips, mouth, esophagus, urinary bladder, prostate, lung, vagina, and cervix, among others. Squamous cells also occur in the lining of the digestive tract, lungs, and other areas of the body. Squamous cell carcinomas are generally treated by surgical excision. Non-surgical options for the treatment of cutaneous SCC include topical chemotherapy, topical immune response modifiers, photodynamic therapy (PDT), radiotherapy, and systemic chemotherapy. The use of topical therapy, such as Imiquimod cream and PDT is generally limited to premalignant and in situ lesions. Radiation therapy is a primary treatment option for patients in whom surgery is not feasible and is an adjuvant therapy for those with metastatic or high-risk cutaneous squamous cell carcinoma. At this time, systemic chemotherapy is used exclusively for patients with metastatic disease.

Until recently, cancer chemotherapy has focused primarily on targeting DNA, microtubule disruption, or on targeting critical cellular proteins or metabolites involved in DNA synthesis and repair. The goal of such pharmacological intervention has been to cause lethal damage to malignant cells with tolerable toxicity to normal cells and organs. Cancer drug development is now turning toward potentially more selective approaches to inducing tumor cell death or cytostasis, and signal transduction inhibitors have advanced from clinical trials to finding approved therapeutic indications.

Hypertrophic scar formation of full-thickness skin wounds or hypertrophic scars and keloids are important problems, against which clinicians are confronted. These scars cause both subjective and objective problems and treatment results are unsatisfactory. Hypertrophic scars are associated with some local wound factors such as prolongation of wound healing, local wound infection, and mechanical wound tension. Hypertrophic scars are frequently associated with surgical injury like sternal incision scars following cardiac surgery and third degree bums. Development of proliferative scars (hypertrophic scars and keloids) has been shown to be associated with abnormalities affecting wound healing steps like cellular proliferation and migration, inflammation, cytokine synthesis and expression, extracellular matrix synthesis and deposition, and maturation. Further, in domestic animals, skin scarring after burn injury is a major medical problem. The recovery of skin function is the concern of burn surgeon in the treatment of skin burn by burn surgeon. However the split-skin graft treatment of full-thickness skin defects leads to scar formation, which is often vulnerable and instable.

Current treatments of hypertrophic scars and keloids are empirical, unreliable and unpredictable. The dermal regeneration usually fails in full-thickness skin loss, resulting in scar formation and wound contraction. Until now there are no prescription drugs for the prevention or treatment of scarring in full-thickness skin loss. Some methods like silicone gel sheeting scar massage, pressure and casting, Imiquimod cream and onion extract have been used for the prevention of hypertrophic scar development in burn patients. However these methods are found ineffective most of time.

Skin thermal injury or skin bums induce body's reaction, which is much more than an initial, local inflammatory response. The burn wound is a continuous, severe threat against the rest of the body due to invasion of infectious agents, antigen challenge and repeated additional trauma caused by wound cleaning and excision. The induced inflammatory response involves a cascades complex of mediators, which control blood supply and microvascular permeability in the wound. Many of these mediators are either part of, or a result of, the inflammatory process. They are responsible for the increased vessel permeability and hydrostatic pressure leading to burn edema and arise from inflammation within a burn wound.

Attempts to suppress the inflammatory reaction by different drugs, have, however, been less successful. Extensive thermal injury and sepsis also results in immunosuppression. The defects causing immunosuppression are still very much under consideration as well as the increasing interest in the control of the inflammatory reactions by cytokines.

Cutaneous barrier injury opens the door to numerous complications such as bacterial infection or fluid loss. Infection of the skin and skin structures frequently occur in surgical wounds and bums. Infection is one of the most serious complications in burn patients, and in particular by Pseudomonas aeruginosa, a Gram - negative rods, an opportunistic pathogen found along with other Pseudomonas spp as part of the normal flora of the human skin. The treatment of patients infected by Pseudomonas aeruginosa is particularly problematic because this organism is inherently resistant to many drug classes and is able to acquire resistance against all effective antimicrobial drugs. Skin infections are responsible for significant human mortality and morbidity and often result in prolonged hospitalization and/or increased health care costs. Topical antimicrobial therapy, such as silver sulfadiazine, to control microbial colonization and subsequent proliferation remains one of the most important methods of burn wound care. And orally administered antibiotics, except the fluoroquinolones, are generally ineffective against most serious skin and soft tissue infections by P. aeruginosa. In patients suffering from burn who develop a pseudomonas aeruginosa septicaemia, the mortality rate is more than 75 %.

Zinc is an extremely common element found in many metal objects and alloys. Zinc is also used in many drugs from toothpaste and sunscreens to medicine using nanotechnology for drug delivery, and occurs naturally in many foods, especially meats. A small amount of zinc is vital for good health. However, although zinc allergies are extremely rare, they are cause for serious concerns. Thus it is preferable to avoid any zinc trace in a medicine, in particular, for compositions being intravenously or orally administered and when zinc is not an active ingredient.

Accordingly, there is a strong need for pharmaceutical compositions having low side-effects for mild and less aggressive treatment of diseases such as squamous cell carcinoma, skin disorders, skin burns, infections of skin wounds and burn wounds by pseudomonas aeruginosa, said compositions which do not comprise metallic chemical such us zinc oxide (ZnO), are based on a natural, abundant product and are inexpensive.

The above objectives are achieved by using a pharmaceutical composition comprising at least 20% or from 20% to 25% of milk fat globules (MFGs) for the topical, injectable or orally administrable treatment of squamous cell carcinoma, skin burns, infections of skin wounds and burn wounds by pseudomonas aeruginosa

The use of MFGs as a mere carrier is already known. EP 0306971 discloses the use of MFGs as a lipid matrix for the delivery of liposoluble active drugs. However the use of MFGs as active ingredient, namely a drug, is not disclosed.

EP 1453526 discloses the use of MFGs in pharmaceutical compositions for the topical treatment of skin disorders, skin bums and skin wounds. Said compositions are an oil-in-water emulsions containing ZnO and further antibiotic, bactericidal drug, sulphonamide or a retinoid.

MFGs, nutritious substances, consist of 99% triglycerides. The triglyceride core of the MFGs is surrounded by a lipid bilayer containing integral membrane proteins.. Milk fat globules consist of 99 % triglycerides, which are synthesized in the mammary gland secretory cell from blood precursors such as glucose, acetate, and low-density lipoproteins. Two thirds of triglyceride core are composed of polysaturated fatty acids (Butyric acid, caproic acid, caprylic acid, capric acid, lauric acid, myristic acid, palmitic acid, stearic acid, and arachidic acid). Polyunsaturated fatty acids are considered essential fatty acids which are not synthesized in human body. The one third remaining of triglycerides is polyunsaturated fatty acids (oleic acid, linoleic acid, linolenic acid and arachidonic acid). Each of these fatty acids is attached to a glycerin. The triglyceride core of the MFGs is surrounded by a lipid bilayer containing integral membrane proteins. The milk fat globules have a diameter range from 1 to 10µm surrounded by a thin membrane called milk fat globule membrane (MFGM). This membrane, which is approximately 10nm thick in cross-section, consists of a complex mixture of proteins, phospholipids, glycoproteins, lactadherin, annexin V, RGD, cholesterol, enzymes, carotenes, vitamins and other minor components (Hamosh M, Peterson JA, Henderson TR, Scallan CD, Kiwan R, Ceriani RL, Armand M,Mehta NR, Hamosh P. Protective function of human milk: the milk fat globule. Semin Perinatol.1999 Jun; 23(3):242-9. Peterson JA, Scallan CD, Ceriani RL, Hamosh M. Structural and functional aspects of three major glycoproteins of the human milk fat globule membrane. AdvExp Med Biol.2001; 501:179-87).

MFGs comprise conjugated linoleic acid, sphingomyelin, butyric acid, ether lipids, β-carotene and vitamins A, D and E, BRAC1 and BRAC2 proteins and other components, which are believed by the inventor to be able to inhibit carcinogenesis in animal models and in various human cancer cell lines.

In different studies, RGD peptides were shown to inhibit the adhesion of cells such as fibroblast to the ECM proteins (e.g., fibronectin, fibrinogen) through its interaction with integrins (αᵥβ₃ and αᵥβ₅) and thus to prevent DNA synthesis and proliferation of primary human fibroblast.

So far, several physiological functions of annexin V have been reported such as control of blood coagulation and can regulate inflammation by inhibiting Phospholipase A₂ (PLA2) activity and therefor inhibiting the conversion of phospholids into arachidonic acid involved in the skin thermal injury. Arachidonic acid is the main precursor for the biosynthesis of both cyclooxygenase and 5-lipooxygenase which include prostaglandins E₂ (PGE₂), prostacyclins, thromboxane B₂ and leukotrienes B₄, the plasma level of which are elevated postburn.

It was demonstrated that the antimicrobial action of MFGs depends on the presence of lipase to release the free fatty acids (FAs) and monoglycerides (MGs). Therefore, the antimicrobial properties of the partially hydrolysed milk fat vary with the composition FAs and MGs.

### Summary of Invention

In a first aspect, the present invention provides a pharmaceutical composition comprising from 20 to 40 wt% of milk fat globules (MFGs) and at least one added C₁₂-C₂₀ fatty acid, and being free of added zinc oxide or added zinc for use in the treatment of skin disorders selected from skin wounds and skin burns, wherein skin wounds and/or skin burns are infected by pseudomonas aeruginosa and/or of cell abnormal proliferation disorders selected from squamous cell carcinoma.

In an aspect, the present invention provides a pharmaceutical composition comprising at least 20 wt% of MFGs and at least one C₁₂-C₂₀ fatty acid, and being free of zinc oxide for treating skin disorders and/or cell abnormal proliferation disorders.

In a further aspect, the invention also provides a method for preparing the above pharmaceutical composition.

Further aspects and preferred embodiments of the present invention are detailed in the appended claims.

### Brief Description of the Drawings

Figure 1 (A) shows squamous cell carcinoma induced by 7,12-Dimethylbenzanthracene (DMBA) in Syrian hamster; (B) to (D) show histological slides of squamous cell carcinoma of Figure 1 (A) with papillary projection into the connective tissue.
Figure 2 shows squamous cell carcinoma induced by DMBA in Syrian hamsters (A) being group of control animals, (B) group of placebo animals, (C) group of treated animals with the pharmaceutical composition of the invention, (D) group of animal not treated by DMBA.
Figure 3 shows a hypertrophic scar tissue formation on the ventral side of the rabbit ear after 28 days post-operation in (A) group of placebo animals, (B) group of treated animals with the pharmaceutical composition of the invention.
Figure 4 shows histologic sections of a hypertrophic scar tissue formation on the ventral side of the rabbit ear after 28 days post-operation (A) group of placebo animals, (B) group of treated animals with the pharmaceutical composition of the invention.
Figure 5 shows the measurement of the release of arachidonic acid metabolites of rabbits skin bums 2 hours after the administration of a saline solution (control), of a placebo (placebo) and the pharmaceutical composition of the invention (trial), (A) measure of prostaglandine E₂, (B) measure of thromboxane B₂, (C) measure of leukotrienes B₄.

### Detailed Description of the Preferred Embodiments

The present invention relates to a pharmaceutical composition comprising at least 20 wt% of milk fat globules (MFGs) and at least one C₁₂-C₂₀ fatty acid, and being free of zinc oxide for treating skin disorders and/or cell abnormal proliferation disorders.

Weight % (wt%) means percentage by weight of the composition.

According to one embodiment, the composition comprises an amount of MFGs from 20 to 40 wt%, from 20 to 35 wt%, from 20 to 30 wt%, preferably from 20 to 25 wt%. No zinc or zinc oxide is added on the contrary of the pharmaceutical composition presented under the form of a cream in EP 1453526. The amount of at least 20 wt% of MFGs in the composition is sufficient to provide an efficient treatment of skin disorders and/or cell abnormal proliferation without the presence of zinc oxide as additional protective, preservative or anti-infectious medium or as an additional active ingredient. In particular, the presence of zinc or zinc oxide shall be avoid for a non-topical administration of a pharmaceutical composition, namely for an administration through intravenous, intramuscular, or orally.

The MFGs may be obtained from butter oil for human consumption, which is butterfat melted and clarified or from ghee, being a semifluid clarified butter having 100% MFGs. Said MGFs have a size range from 1 to 10 µm.

The C₁₂-C₂₀ fatty acid is selected from the group consisting of stearic acid, oleic acid, isostearic acid, linoleic acid, cetearic acid, myristic acid. Preferably, said C₁₂-C₂₀ fatty acid is selected from the group consisting oleic acid and cetearic acid. Most preferably, said C₁₂-C₂₀ fatty acid is oleic acid or cetearic acid. Said C₁₂-C₂₀ fatty acid is present in an amount of 10 wt%. Said C₁₂-C₂₀ fatty acid provides a structure as thickener to the galenic preparation of the pharmaceutical composition of the invention and may also provide a synergistic effect in the treatment of skin bums with the active ingredient, namely MFGs.

The composition further comprises an emulsifier. It forms an oil-in-water emulsion together with the emulsifier, wherein the MFGs are added as active ingredient. The pharmaceutical composition may be administered under the form of a suspension (Syrian suspension) or under the form of a cream (Syrian cream). The choice of the form of the preparation depends on the way of the administration, which may be intravenous, intramuscular, oral or topic. Thus a cream in form of an oil-in-water emulsion is selected as a drug of choice for a topic administration, in particular in patients suffering from burn or thermal injury. In general topic ointment preparations are less acceptable to patients than cream formulations: cream is perceived to be easier to apply and to cause less garment soiling than ointments.

In a further embodiment, skin disorders comprise skin wounds, skin burns, skin scars, keloids and hypercornification. The pharmaceutical composition of the invention may be also used for preventing the formation of skin scars, hypertrophic skin scars, keloids, said skin disorders associated with abnormalities affecting wound healing steps like cellular proliferation and migration, inflammation, cytokine synthesis and expression, extracellular matrix synthesis and deposition, and maturation. Said prevention of these skin scars, hypertrophic skin scars, keloids, said skin disorders associated with abnormalities affecting wound healing steps may be effected by the topical administration of the pharmaceutical composition of the invention, and in particular, by the topical administration of the pharmaceutical composition under the preparation of a cream(Syrian cream).

In another embodiment, cell abnormal proliferation disorders are selected form squamous cell carcinoma or cancer. Without to be bound by the theory, MFGs comprise several proteins and other components, as among others conjugated linoleic acid, sphingomyelin, butyric acid, ether lipids, β-carotene and vitamins A and D, which are able to inhibit the carcinogenesis in vivo animal models and human cancer cell lines. Thus lactadherin, annexin V present in MFGs inhibit the blood coagulation when topically applied and may inhibit the activation of prothrombin and/or thrombin. Since thrombin is recognized not only to induce venous thrombosis but also tumor growth, metastasis and angiogenesis, MFGs present in a pharmaceutical composition may be used for treating cancer diseases. In the present application, the presence of MFGs in the topical application on the skin thermal injuries provides the inhibition of some arachidonic acid metabolites (PGE₂, TXA₂ and LTB₄). Since said metabolites are involved in numerous cancers, such as cancer of breast, pancreas, prostate, lung, skin, liver, urinary bladder and colon, the pharmaceutical composition of the invention comprising at least 20 wt% MFGs may be used for the treatment of such cancers, for the reduction of carcinogenesis and metastatic capacity of tumor, for the reduction of tumor growth. Said cancers involving cyclo-oxygenase 1 and 2 (COX), lipo-oxygenase (LOX) in the process of abnormal cell proliferation may be treated by a pharmaceutical composition of the invention.

According to another embodiment, the pharmaceutical composition may be used for treating skin wounds and skin burns, in particular, skin wounds and skin bums infected by pseudomonas aeruginosa. The at least 20 wt% MFGs as active ingredient in the pharmaceutical composition of the invention without any additional anti-infectious, antifungal and antimicrobial medicine such as zinc oxide or antibiotics is efficient or as sufficiently efficient as sulfadiazine to treat and prevent the gram-negative rod, pseudomonas aeruginosa, infecting and/or recolonizating wounds and bums in patient, preferably in burned patient. One advantage to use such pharmaceutical composition comprising MFGs without the addition of anti-infectious drug is to avoid drug-resistance by the infectious agent, such as pseudomonas aeruginosa. Moreover such pharmaceutical composition of the invention may be used to treat such skin wounds or skin burn wounds infected by pseudomonas aeruginosa showing drug resistance.

The present invention also relates to a method for treatment of skin disorders and/or cell abnormal proliferation disorders in a subject comprising administering to the subject an effective amount of a pharmaceutical composition comprising at least 20 wt% of milk fat globules (MFGs) and at least one C₁₂-C₂₀ fatty acid, and being free of zinc oxide. Said administration is topical, intravenous, intramuscular or oral.

The present invention further relates to a method for preparing the pharmaceutical composition of the invention under a preparation of a suspension or a preparation of a cream. The form of the preparation, namely the oil-in-water emulsion, depends on the excipient added to obtain one or the other form. Said method comprises a step, wherein the emulsifier is melt in boiling water.

At this step, all compounds soluble in water may be added. The emulsifier may be selected from alkyl alcohols, alkyl polyglucosides, polyglycerol alkyl esters, C₁-C₄ esters of alkyl alcohols, C₁-C₄ esters of alkyl carboxylates, alkyl amides, alkyl betaines, and alkyl phosphates or phospholipids, alkyl quaternary amines, alkyl amine oxides, polyethoxylated alkyl alcohols, alkyl esters of polyethylene glycol, and mixtures thereof. Preferably the emulsifier is tri-ethanol amine.

The method comprises a further step including the melting of the oily material comprising MFGs and fatty acids by heating up to the melting point of said ingredients. The oily material may further comprise oily excipient selected from paraffin, vaseline, isopropyl myristate. Then the method comprises the step of mixing or blending the melted oily material within the melted emulsifier in a mixer at a speed of 1200 rotations/minute for 5 minutes.

The process for making the pharmaceutical composition of the invention and its properties will be better understood by those skilled in the art in view of the following description and exemplification of the formulations and the results of *in vivo* experiments and assays.

### Examples

### Example 1: Syrian suspension

This preparation contains MFGs as active ingredient in form of oil-in-water emulsion is more suitable for an injectable administration (intravenously or intramuscularly) or oral administration. This product is Injectable in IV, IM and oral administration.

The formula of the Syrian suspension is the following:

| | | |
|---|---|---|
| Pure MFGs | 20 g | 20 % |
| Oleic acid | 10 g | 10 % |
| Tri-ethanol amine (Merck) | 2 g | 2 % |
| Distilled water | q.s. 100 g | q.s. 100% |

MFGs used as active ingredient have a diameter from 1 to 10 µm. MFGs come from the market, i.e. from Butter Oil or Pure Butter Fat Ghee, which contains 100 % MFGs. Oleic acid is the preferred fatty acid because its liquid form helps to maintain the suspension liquid.

The suspension may be prepared in the same way describe in EP 0306971. This preparation is processed as follows:
1. The water is put in a mixer, heated up to boiling point. At this stage, the materials, which are able to melt in water such as tri-ethanol amine, are added.
2. The oily materials, MFGs and oleic acid, are heated up to melting point.
3. The melted oily materials are slowly added to the mixture of water. The mixing is continued at a speed of 1200 rotations / minute for about 5 minutes.

The process was carried out under sterile conditions. Thus this sterile suspension is packaged in sterile bottle.

### Example 2: Syrian cream

This preparation under the form of a cream contains MFGs as active ingredient in form of an oil-in-water emulsion and is more suitable for a topical administration. In general, topical ointment preparations are less acceptable to patients than cream formulations: cream is perceived to be easier to apply and to cause less garment soiling than ointments. To enhance patient acceptance, therefore, a new cream formulation of MFGs has been developed.

The formula of the Syrian cream is the following:

| | | |
|---|---|---|
| MFGs | 25 g | 25 % |
| Cetearic acid | 10 g | 10 % |
| Isopropyl myristate | 7 g | 7 % |
| Liquid paraffin | 7 g | 7 % |
| Vaseline | 3 g | 3 % |
| Tri-ethanolamine (Merck) | 2 g | 2 % |
| Distilled water | q. s. 100 g | q.s. 100 % |

MFGs used as active ingredient have a diameter from 1 to 10 µm. MFGs come from the market, i.e. from Butter Oil or Pure Butter Fat Ghee, which contains 100 % MFGs.

The use of cetearic acid is preferred because it gives the creamy form to the preparation. Isopropyl myristate and liquid paraffin synergistically reduce the cream absorption by gauze and prevent gauze adhering on burned area.

The cream may be prepared in the same way describe in EP 0306971. This preparation is processed as follows:
1. The water is put in a mixer, heated up to boiling point. At this stage, the materials Tri-ethanol amine, which are able to melt in water, are added.
2. The oily materials, MFGs, cetearic acid, isopropyl myristate and further with Vaseline and liquid paraffin, are heated up to melting point.
3. The melted oily materials are slowly added to the mixture of water. The mixing is continued at a speed of 1200 rotations / minute for about 5 minutes.

The process is carried out under sterile conditions and after cooling, the cream is filled in tubes. The final cream is tested for any microbes contamination.

### Example 3: Treatment of squamous cell carcinoma induced by DMBA in Syrian hamster

The pharmaceutical composition of the invention, Syrian suspension containing 20% MFGs, is intravenously administered to Syrian hamsters suffering from squamous cell carcinomas in buccal pouch, which are induced by DMBA (7-12-dimethylbenz[a]anthracene).

### Animals experiment

The animals were handled according to the guidelines and recommendations of the Institutional Animal Care and Use Committee (IACUC). The experiment was carried out on 8-10 weeks old male Syrian golden hamsters of 100-110 g.

### Carcinogenesis

In all animals, the right buccal pouches mucosa of hamster were painted three times per week for 14 weeks with a solution of 0.5% DMBA (Sigma Chemical Company, St. Louis, MO, USA) in liquid paraffin with a No. 4 brush.

### Tumours assessments

Before beginning the treatment with the pharmaceutical composition of the invention, the buccal pouch of each animal was inspected and premalignant lesions or tumour development were evaluated. The tumour formation and size were calculated by multiplying the mean tumour volume (4/3·πr³) (r = 1/2 tumour diameter in mm) with the mean number of tumours. Samples of tumours were formalin-fixed and paraffin-embedded and sections thereof were stained with hematoxylin and eosin (H&E) for histopathological analysis. During the administration of the Syrian supension, the tumours in the pouch were macroscopically checked once a week. After the treatment, the tumour incidence, volume and control were examined and calculated.

### Treatment of the animals with the pharmaceutical composition of the invention

The Syrian suspension was intravenously administered through a tube was introduced in the femoral vein of each animal. After randomization, the animals were divided into three groups A, B, and C of ten animals each. In group A, each animal received 0.2 ml of Syrian suspension each day. In group B, each animal received 0.2 ml of base of Syrian suspension lacking MFGs, the active ingredient, daily. In group C, each animal received 0.2 ml of a saline solution each day.

### Histopathological analyses

The whole cheek pouch was excised and flattened on a transparency plate for counting the number of visible tumours. Formalin-fixed pouches were cut into 4-6 pieces of approximately equal width, Swiss-rolled, processed and embedded in paraffin. Thirty sections (5 µm) of each sample were cut and the 1st, 15th and 30th slides were stained with Hematoxylin and Eosin (H&E) for histopathological analysis. Basal cell hyperplasia, dysplasia, squamous cell carcinoma and papillomas were diagnosed using established criteria (Kramer I, Lucas R, Pindborg J, Sobin L. WHO, Collaborating center for oral precancerous lesions: definitions of leukoplakia and related lesions: an aid to studies on oral precancer. Oral Surg. Oral Med. Oral Pathol 1978;46:518-589. Leininger, I.; KJokinen, M. Pathology of tumors in laboratory animals. Turosov, V., editor. Albany, NY: IARC; 1982. p. 167-169)

### Results

### - Before the treatment with Syrian suspension

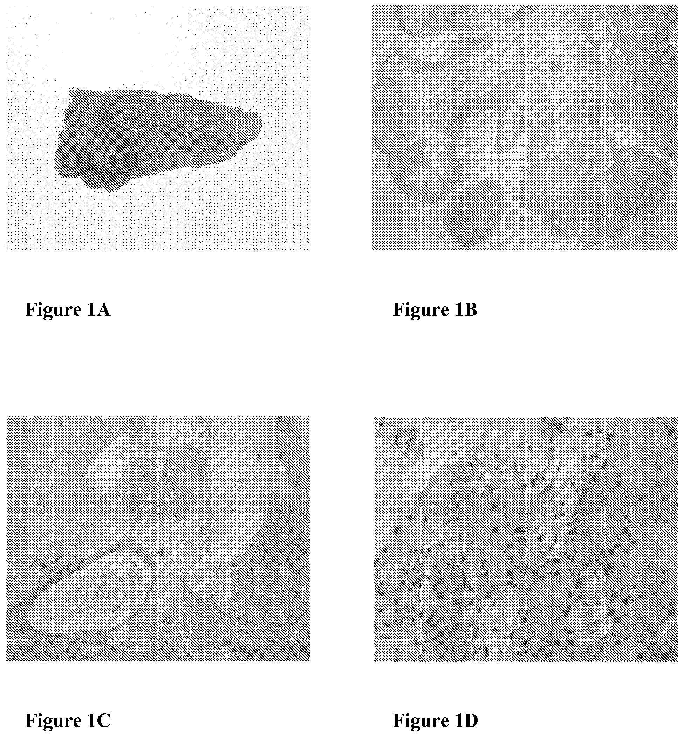

Topical application of DMBA to the hamster buccal pouch (HBP) for 14 week resulted in well-developed large and exophytic tumours in the oral cavity of hamsters and in well-differentiated squamous cell carcinomas. 110% tumour formation with mean tumour volume of 108 mm³ ± 9 mm³ are observed (Figure 1, A). Histologically, HBP tumours induced by DMBA were invasive squamous cell carcinomas with papillary projections of squamous epithelium into the connective tissues (Figure 1B, C and D).

### - After the treatment with Syrian suspension

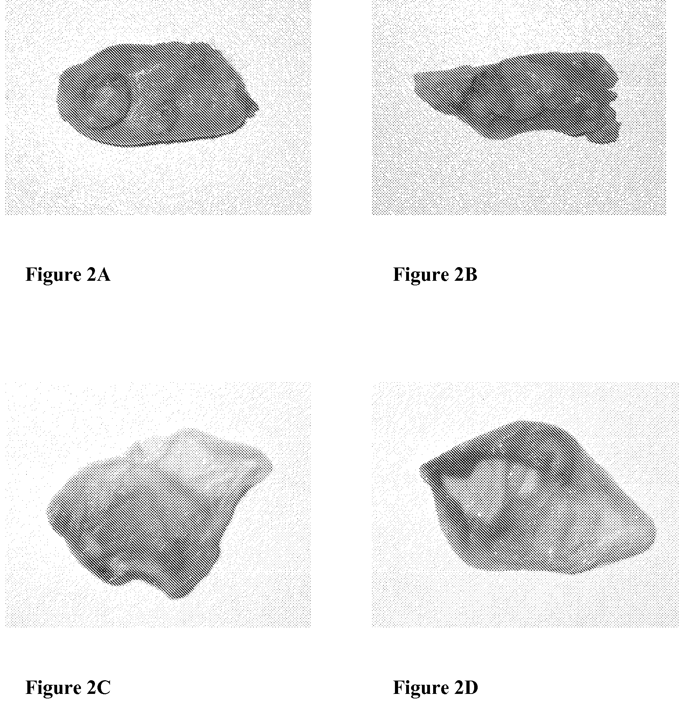

Macroscopically, in the first week, a reduction of about 20% of tumour size was observed in treated group A compared to other groups (groups B and C). In the second week, the inventors also noticed that the tumours color converts from dark color to light red in treated group A compared to control and placebo group (groups B and C, respectively), showing a blood perfusion of the tumor. In the treated group A, the tumours were covered by a rough whitish layer, which was not the case in groups B and C, wherein this layer was absent. The intravenously administration of Syrian suspension significantly reduces the tumour volume. This reduction begins at the third week after the beginning of the treatment. At eight weeks after the beginning of the treatment, the animals treated with Syrian suspension presented no more tumours. Tumours were observed in groups B and C without any changes in the form of tumours (Figure 2). No malignant neoplasms or pre-neoplastic lesions were observed in animals in groups treated with Syrian suspension compare to groups B and C.

According to these results, the inventors suggest that the MFGs concentrate in affected area (tumours), which leads to decrease the tumours volume of 20 % in treated animals in the first week of treatment and to the disappearance of the tumours after 8 weeks of treatment in Syrian hamster. Therefore, the inventors demonstrate that the Syrian suspension, a pharmaceutical composition of the invention is efficient to treat these type of tumours being a model of squamous cell carcinoma.

### Example 4: Treatment of hypertrophic scars in animal ear model

The pharmaceutical composition of the invention, Syrian cream containing 25% MFGs, is topically administered to New Zealand male rabbits suffering from hypertrophic scars induced on the rabbit's ear, being a model for human hypertrophic scars.

### Animals study

Hypertrophic scars were induced on the ear of 10 New Zealand male rabbits, weighing 2.5-3.0 kg according to the hypertrophic scar ear model described by Moris et al. (Morris DE, Wu L, Zhao LL, Bolton L, Roth SI, Ladin DA, Mustoe TA. Acute and chronic animal models for excessive dermal scarring: quantitative studies. PlastReconstr. Surg. 1997 Sep; 100(3):674-81). The animals were handled according to laboratory conditions and veterinary supervision and the guidelines and recommendations of the Institutional Animal Care and Use Committee (IACUC).

### Induction of hypertrophic scars by wound injuries

Four circular full-thickness skin of 7 mm diameter were excised up to bare cartilage on the ventral surface of each ear by using microsurgical. The removal of the perichondrial layer delays the epithelialization of the wound, which induces hypertrophic scar.

### Treatment with the pharmaceutical composition of the invention

The animals were divided into two groups of five each. In the first group, the Syrian cream containing 25% MFGs was applied four times daily onto the wound for 28 postoperative days. In the second group, the Syrian cream without active ingredient (placebo) was applied four times daily onto the wound for 28 postoperative days. Rabbits were sacrificed and the scars were completely excised with a thin rim of normal surrounding tissue at the 28^{th} postoperative day 28. The scars were fixed in 10% formalin and sections thereof were hematoxylin-eosin stained for histopathological and histomorphometric analyses. Scar hypertrophy index (SHI) was calculated by dividing the total scar area (hypertrophic area plus baseline) by the baseline area with the help of histomorphometric examination for all scars. Comparisons were made between unwounded skin and other groups using study was used student's t-test to compare the means between study groups. The level of significant was set to P values less than 0.05. Data are presented as mean ± SEM of the SHI.

### Results

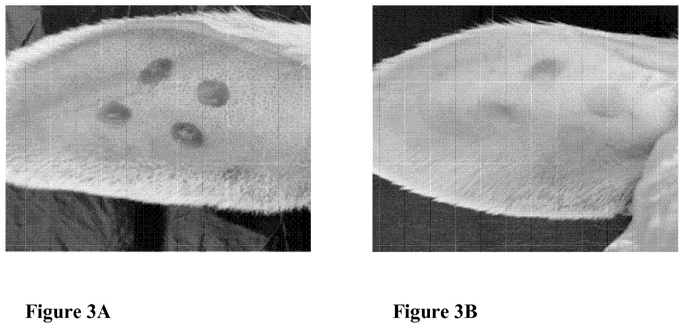

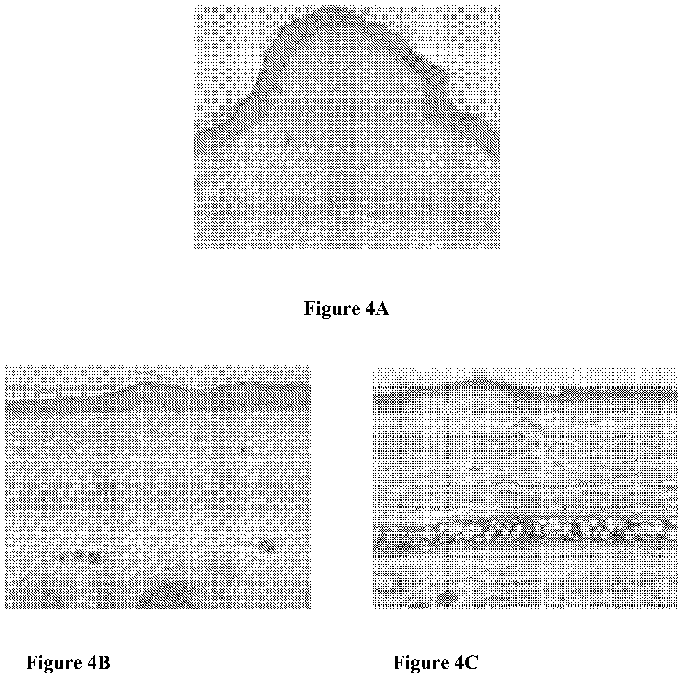

The inventors noticed that the application of the Syrian cream containing the MFGs cream inhibited the blood postoperative coagulation compared with the placebo group. There was no crust formation in this first group and the wounds were covered by a whitish surface film after three days up to the healing of the wounds. In placebo group, a crust was formed after four days. Although the wounds in the placebo group healed 14 days faster than in the group, wherein the Syrian cream with 25% MFGs was topically applied, only in this treated group, the formation of hypertrophic scars was prevented and no erythematous, hypertrophie and larger wound was observed on the contrary what was observed in the placebo group (Figure 3 A and B). In the group of the animals treated with the pharmaceutical composition comprising 25% MFGs, the mean hypertrophic scar index (SHI) was 1.08 ± 0.01 compared with the placebo groups, wherein the SHI was 1.54 ± 0.03 (P = 0.03) (Figure 4). This corresponds to a reduction of hypertrophic scar due of 85% in the treated group to the inhibition of the synthesis of extra-cellular matrix, and in particular collagen. Thus the inventors show that MFGs prevent hypertrophic scar formation, when applied under the form of cream. The inventors also suggest that the pharmaceutical composition of the invention may be efficient to other fibro proliferative disorders involving an excessive accumulation of collagen.

### (Reference) Example 5: Treatment of wounds and burns

The pharmaceutical composition of the invention, Syrian cream containing 25% MFGs, is topically administered to New Zealand male rabbits suffering from burn wounds to decrease the inflammation and treat bums and thermal injury.

### Animals study

Thirty male New Zealand white rabbits weighting 2-2.5 kg were used in this experiment. The animals were handled according to laboratory conditions and veterinary supervision and the guidelines and recommendations of the Institutional Animal Care and Use Committee (IACUC).

### Induction of burn wounds and thermal injury

The aluminum stamp described by Knabl et al. (Controlled partial skin thickness bums: an animal model for studies of burn wound progression. Bums 1999;25:229-35) was modified. An electronic temperature controller with a thermocouple type feedback sensor was added in order to allow precise optimal temperature monitoring at the burning surface of the stamp. A dimmer was also integrated to provide further temperature control. Continuous confirmation and monitoring of the aluminum stamp temperature was hence attained. The round stamp of 2.2 cm diameter was fitted to the ordinary soldering iron producing a burn area of approximately 3.8 cm². The desired stamp temperature of 100°C was reached 15 min after switching on the electric current. It was then applied for 12 s to produce a full-thickness skin burn.

### Treatment with the pharmaceutical composition of the invention

The burned animals were randomly divided into three equal groups. Each group consists of 10 animals. In the group A, all animals were treated with 2 g Syrian cream on the injured area. In the group B, all animals were left as control covered by simple saline and in the group C (placebo group), all animals were treated with 2 g of Syrian cream lacking MFGs. Subsequently burned areas in all animals were covered with an occlusive dressing (Tegaderm, 3M, USA) during 2 h postburn.

### Measurement of the inhibition of arachidonic acid metabolites

Two hours after applying the treatment, approximately 2 cm² skin biopsies were taken from burned skin includ-ing dermis and epidermis. Biopsies were rinsed in saline at 38°C and used to measure arachidonic acid metabolites: Prostaglandin E₂ (PGE₂), Thromboxane B₂ (TXB₂) and Leukotrienes B₄ (LTB₄) by Elisa assay (Cayman Chemical Company, UK,). Since TXB₂ is unstable, they are usually quantified by measuring their stable metabolite TXA₂. Statistical analysis was performed using analysis of variance (ANOVA). Contrasts were formed at 95% confidence intervals with subsequent analysis of significance by Sheffe's test. Data are mean ± 1.96*SEM. The analysis was carried out using (Analytical software 1998) vergin2.0.

### Results

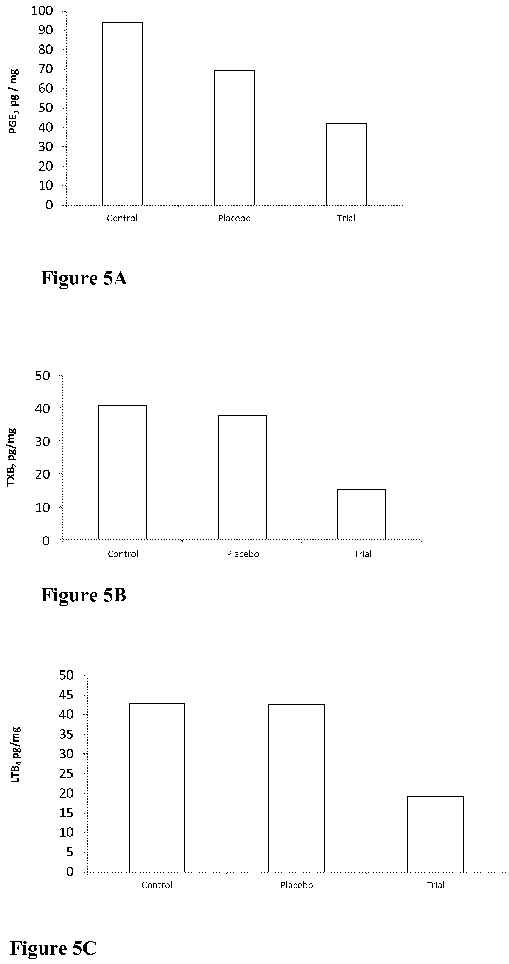

In burned skin, topical treatment with the Syrian cream containing 25 % MFGs significantly inhibited the release of PGE₂ (P = 0.0000) (Figure 5A), TXB₂ (P = 0.0000) (Figure 5B) and LTB₄ (P = 0.0000) (Figure 5C) compared with the control and placebo groups. These results shows that the topical treatment of burn wounds of thermally injured skin with a pharmaceutical composition with MFGs as active ingredient inhibits the release of arachidonic acid metabolites. Said metabolites are mediators either part of or result of the inflammatory process of burn wounds. They are responsive for the increased vessel permeability and hydrostatic pressure leading to burn oedema. It is published that PGE₂ appear to be one of the more potent inflammatory mediators causing postburn vasodilation in wounds, which, when coupled with the increased microvascular permeability, contributes to oedema. It is also known that prostaglandins are involved in the pathogenesis of pain and fever in inflammation. It is shown that specific antiprostaglandins may be effective in preventing these progressive changes and in improving dermal perfusion in animals leading to decrease the depth and the amount of skin necrosis after burn. In previous studies, burn treatments with various thromboxane inhibitors improved postburn dermal microcirculation, decreased postburn oedema, improved burn wound healing and improved skin perfusion in full-thickness bums. In further scientific publications, it is also demonstrated that the blockade of LTB₄ synthesis or LTB₄ receptors results in reduced neutrophil migration to the mice peritoneal cavity in case of sepsis, in inhibition of neutrophils and eosinophils infiltration and in exhibiting antinociceptive effect of inflammatory pain in paw biopsies in mice and rat.

Accordingly, these results of this experiment shows that a pharmaceutical composition comprising at least 20% MFGs as active ingredient is efficient to treat burn wounds by inhibiting arachinidonic acid metabolites PGE₂, TXB₂ and LTB₄ after the topical administration of said composition under cream form.

### Example 6: Treatment of wounds and burns infected by Pseudomonas aeruginosa

The pharmaceutical composition of the invention, Syrian cream containing 25% MFGs, is topically administered to Wistar male rats suffering from burn wounds infected by Pseudomonas aeruginosa. Topical antimicrobial therapy remains one of the most important methods of burn wound care. The aim of topical antimicrobial therapy is to control microbial colonization and subsequent proliferation. Today, silver sulfadiazine (SSD) is the most widely used topical antimicrobial agent.

### Animals study and infection by P. aerugionsa

Thirty-two male male Wistar rats weighing 200 to 220 g were used and handled according to laboratory conditions and veterinary supervision and the guidelines and recommendations of the Institutional Animal Care and Use Committee (IACUC).After being anesthetized in the rats backs were shaved. Using a standard method, a full-skin thickness dorsal scald burn was caused on approximately 15% of the each rat's body surface by placing them in boiling water. The animals were resuscitated with an intraperitoneal injection of 2 ml of lactated Ringer's solution. Ten minutes after the burn, each animal was seeded with 0.5 ml of broth containing 1 × 108 colony-forming units of P. aeruginosa (ATCC 27853) by swabbing. The animals were placed in separate sterilized cages and allowed to recover.

### Studies of antimicrobial activity

After 24 hours, the animals were randomly divided into four groups eight animal each. In group one, the Syrian cream preparation containing 25% MFGs were topically applied to the burn wound the animals twice a day. In the group two, placebo group, the animals were topically treated with the Syrian cream base preparation, which does not comprise any MFGs as active ingredient, twice a day y to the burn wound. In the group three, the animals were treated with Silver sulfadiazine 1% (FlammazineTM Smith & Nephew), twice a day. And in the group four, control group, 0.7ml of a solution of 0.9% sodium chloride was topically applied on burned and infected animals. Sterile gauzes were placed over these areas, and all the dressing were attached with the skin staplers.

All the animals were anesthetized and killed after 7 days after burn injury. All cultures were obtained using an aseptic technique. Initially, thoracotomy was performed. Blood cultures were obtained from the left ventricle, and lung biopsies were obtained. Blood specimens were placed in brain heart infusion broth; both were incubated at 35°C degrees and isolated organisms were identified by standard methods.

Full-skin thickness punch biopsies of 9 mm were obtained from the center of the burn eschar. After removal of eschar and underlying fascia, a separate biopsy of paravertebral muscle deep to the burn eschar was obtained. Separate quantitative cultures of eschar and muscle were performed using a standard method (Ülkür, Ersin MD; Öncül, Oral MD; Karagöz, Hüseyin MD; Çeliköz, Bahattin MD; Çavuslu, Saban MD. Comparison of Silver-Coated Dressing (Acticoat™), Chlorhexidine Acetate 0.5% (Bactigrass®), and Silver Sulfadiazine 1% (Silverdin®) for Topical Antibacterial Effect in Pseudomonas Aeruginosa-Contaminated, Full-Skin Thickness Burn Wounds in Rats. Journal of Burn Care & Rehabilitation. Issue: Volume 26(5), September/October 2005, pp 430-433. Herndon DN, editor. Herndon's total burn care. 2nd ed. London: W.B. Saunders Co.; 2001. p. 98-169).

### Results

No animal deaths were recorded throughout the experimental protocol. The frequency of recovery of the seeded organisms from each culture site is detailed in Table 1. The swabbed organism was recovered from control and placebo groups only.

**Table 1: Frequency of recovery of seeded organisms from each culture site**

| Animals Group | AnimalSeeds | Eschar | Muscle | Blood | Lung |
|---|---|---|---|---|---|
| Group IV: Control | 8 | 8 | 8 | 6 | 6 |
| Group II: Placebo | 8 | 10 | 8 | 8 | 8 |
| Group III: SSD | 8 | 0 | 0 | 0 | 0 |
| Group I: MFGs | 8 | 0 | 0 | 0 | 0 |

As a result, the Syrian cream containing 25 wt% MFGs is an efficient antipseudomonal agent. Pseudomonas species play a prominent role as etiologic agents in serious infections in burned patients. When the host is immunocompromised, as in the case of a thermal burn or surgical wound, this opportunistic bacterium can quickly colonize and infect the burn and wound sites Effective therapy of burn wound infections requires the additional expense of parentally administered broad-spectrum antibiotics. In this study the pharmaceutical preparation comprising MFGs, without additional antibiotics, is efficient and may be used also for the prevention and the treatement of the infection of burn wound.

## Claims

1. A pharmaceutical composition comprising from 20 to 40 wt% of milk fat globules (MFGs) and at least one added C₁₂-C₂₀ fatty acid, and being free of added zinc oxide or added zinc for use in the treatment of skin disorders selected from skin wounds and skin burns, wherein skin wounds and/or skin burns are infected by pseudomonas aeruginosa and/or of cell abnormal proliferation disorders selected from squamous cell carcinoma.

2. The composition for use according to claim 1, wherein the amount of MFGs is from 20 to 25 wt%.

3. The composition for use according to anyone the preceding claims, wherein MFGs have a size range from 1 to 10 µm.

4. The composition for use according to anyone of the preceding claims, wherein said at least one added C₁₂-C₂₀ fatty acid is selected from the group consisting of stearic acid, oleic acid, isostearic acid, linoleic acid, cetearic acid, myristic acid.

5. The composition for use according to anyone of the preceding claims, wherein the amount of the at least one added C₁₂-C₂₀ fatty acid is 10 wt%.

6. The composition for use according to anyone of the preceding claims, wherein said composition further comprises an emulsifier and is an oil-in-water emulsion of MFGs being the active ingredient.

7. The composition for use according to anyone of the preceding claims, wherein the composition may be administered topically, intravenously, intramuscularly, or orally.

8. The composition for use according to anyone of the preceding claims further comprising one or more optional oily excipients, wherein the oily excipient is selected from paraffin, vaseline, isopropyl myristate

9. The composition for use according to anyone of claims 6 to 8, wherein the emulsifier is selected from alkyl alcohols, alkyl polyglucosides, polyglycerol alkyl esters, C₁-C₄ esters of alkyl alcohols, C₁-C₄ esters of alkyl carboxylates, alkyl amides, alkyl betaines, and alkyl phosphates or phospholipids, alkyl quaternary amines, alkyl amine oxides, polyethoxylated alkyl alcohols, alkyl esters of polyethylene glycol, and mixtures thereof.

## Patentansprüche

1. Pharmazeutische Zusammensetzung, die 20 bis 40 Gew.% Milchfettkügelchen (MFGs) und mindestens eine zugefügte C₁₂-C₂₀-Fettsäure umfasst und frei von zugefügtem Zinkoxid oder zugefügtem Zink ist, zur Verwendung in der Behandlung von Hauterkrankungen ausgewählt aus Hautwunden und Hautverbrennungen, wobei Hautwunden und/oder Hautverbrennungen durch Pseudomonas aeruginosa infiziert sind, und/oder abnormalen Zellproliferationserkrankungen ausgewählt aus Plattenepithelkarzinom.

2. Zusammensetzung zur Verwendung nach Anspruch 1, wobei die Menge der MFGs 20 bis 25 Gew.% beträgt.

3. Zusammensetzung zur Verwendung nach einem der vorhergehenden Ansprüche, wobei die MFGs einen Größenbereich von 1 bis 10 µm haben.

4. Zusammensetzung zur Verwendung nach einem der vorhergehenden Ansprüche, wobei die mindestens eine zugefügte C₁₂-C₂₀-Fettsäure ausgewählt ist aus der Gruppe bestehend aus Stearinsäure, Ölsäure, Isostearinsäure, Linolsäure, Cetylstearinsäure, Myristinsäure.

5. Zusammensetzung zur Verwendung nach einem der vorhergehenden Ansprüche, wobei die Menge der mindestens einen zugefügten C₁₂-C₂₀-Fettsäure 10 Gew.% beträgt.

6. Zusammensetzung zur Verwendung nach einem der vorhergehenden Ansprüche, wobei die Zusammensetzung ferner einen Emulgator umfasst und eine Öl-in-Wasser-Emulsion ist, bei der MFGs der aktive Bestandteil sind.

7. Zusammensetzung zur Verwendung nach einem der vorhergehenden Ansprüche, wobei die Zusammensetzung topisch, intravenös, intramuskulär oder oral verabreicht werden kann.

8. Zusammensetzung zur Verwendung nach einem der vorhergehenden Ansprüche, die ferner einen oder mehrere optionale ölige Hilfsmittel umfasst, wobei das ölige Hilfsmittel ausgewählt ist aus Paraffin, Vaseline, Isopropylmyristat.

9. Zusammensetzung zur Verwendung nach einem der Ansprüche 6 bis 8, wobei der Emulgator ausgewählt ist aus Alkylalkoholen, Alkylpolyglucosiden, Polyglycerinalkylestern, C₁-C₄-Estern von Alkylalkoholen, C₁-C₄-Estern von Alkylcarboxylaten, Alkylamiden, Alkylbetainen und Alkylphosphaten oder Phospholipiden, quaternären Alkylaminen, Alkylaminoxiden, polyethoxylierten Alkylalkoholen, Alkylestern von Polyethylenglykol und Mischungen davon.

## Revendications

1. Composition pharmaceutique comprenant de 20 à 40 % en poids de globules de matière grasse de lait (MFG) et au moins un acide gras en C₁₂-C₂₀ ajouté, et étant exempte d'oxyde de zinc ajouté ou de zinc ajouté pour utilisation dans le traitement de troubles de la peau choisis parmi des plaies cutanées et des brûlures cutanées, les plaies cutanées et/ou les brûlures cutanées étant infectées par Pseudomonas aeruginosa et/ou de troubles de prolifération cellulaire anormale choisis parmi un carcinome épidermoïde.

2. Composition pour utilisation selon la revendication 1, dans laquelle la quantité de MFG est de 20 à 25 % en poids.

3. Composition pour utilisation selon l'une quelconque des revendications précédentes, dans laquelle les MFG ont une plage de taille de 1 à 10 µm.

4. Composition pour utilisation selon l'une quelconque des revendications précédentes, dans laquelle ledit au moins un acide gras en C₁₂-C₂₀ ajouté est choisi dans le groupe constitué de l'acide stéarique, l'acide oléique, l'acide isostéarique, l'acide linoléique, l'acide cétéarique, l'acide myristique.

5. Composition pour utilisation selon l'une quelconque des revendications précédentes, dans laquelle la quantité de l'au moins un acide gras en C₁₂-C₂₀ ajouté est de 10 % en poids.

6. Composition pour utilisation selon l'une quelconque des revendications précédentes, ladite composition comprenant en outre un émulsifiant et étant une émulsion huile dans eau de MFG étant la substance active.

7. Composition pour utilisation selon l'une quelconque des revendications précédentes, la composition pouvant être administrée par voie topique, par voie intraveineuse, par voie intramusculaire ou par voie orale.

8. Composition pour utilisation selon l'une quelconque des revendications précédentes comprenant en outre un ou plusieurs excipients huileux, l'excipient huileux étant choisi parmi la paraffine, la vaseline, le myristate d'isopropyle.

9. Composition pour utilisation selon l'une quelconque des revendications 6 à 8, dans laquelle l'émulsifiant est choisi parmi des alcools alkyliques, des alkyl-polyglucosides, des esters d'alkyle de polyglycérol, des esters en C₁-C₄ d'alcools alkyliques, des esters en C₁-C₄ de carboxylates d'alkyle, des alkylamides, des alkylbétaïnes, et des alkyl-phosphates ou phospholipides, des alkylamines quaternaires, des oxydes d'alkylamine, des alcools alkyliques polyéthoxylés, des esters d'alkyle de polyéthylène glycol, et des mélanges de ceux-ci.
